# EUROPEAN PATENT APPLICATION

(11) **EP 1 762 272 A1**
(43) Date of publication of application: **14.03.2007**
(21) Application number: 05076641.9
(22) Date of filing: 18.07.2005
(51) Int. Cl.: A61Q 19/00, A61Q 13/00, A61K 8/34, A61K 8/97

(54) **Use of vinic alcohol in personal care products, cosmetics and perfumes.**

(71) Applicant: Botica Comercial Farmacêutica Ltda., Sâo José dos Pinhais PR 830 65-260 (BR)
(72) Inventor: Feferman, Israel Henrique S., Sao Jose Dos Pinhais PR (BR); Veiga, Cesar Antônio, Curitiba PR (BR)
(74) Representative: Carvajal y Urquijo, Isabel

(57) **Abstract**

This invention relates to the use of a vinic alcohol in cosmetic compositions, perfumes and personal care products. The vinic alcohol is characterized as being a product from the grape fermentation followed by a purification by distillation and is used in this invention in total or partial substitution of the conventional ethylic alcohol. The vinic alcohol contains residual substances, which are a characteristic feature of the fermentation of grapes, affording a characteristic odor to the ethanol, contributing to a different olfactory sensorial perception.

## Description

This invention deals with the use of a specific type of ethylic alcohol, the vinic alcohol, in personal care products, cosmetics and perfumes, with a view to affording drying properties, volatility, astringency, cleaning, solubility of other ingredients, antimicrobial action, inter alias. The use of this invention results in a different product, owing to the characteristic odor of the vinic alcohol and to its contribution to the final olfactory sensorial perception.

The cosmetic preparations, the personal care products and the perfumes represent a class of products for affording cleaning protection, treatment, coloring, fragrance, and deodorization, among other benefits of the skin, mucous membranes and hair.

Cosmetics are products for an external use, intended for protecting or embellishing the different parts of the body, such as creams and beauty lotions, hand creams and alike, facial masks, milky or creamy solutions, astringents, hand lotions, make-up, cosmetic oil, anti-solar preparations, sun-tanning preparations and alike, capillary dyes, hair-coloring agents, preparations for waving and fixing hair, hair spays, brilliantine and alike, capillary lotions, depilatories, nail preparations and alike.

Personal care products are understood to be products for external use, anti-septic or not, intended for the neatness or for the corporal decontamination, comprising soaps, shampoos capillary fixatives, dentifrice, mouth rinsing, antiperspirant ingredients. deodorants, shaving products and after-shaving, inter alias.

As perfumes, they are understood to be products of an aromatic composition, obtained from natural or synthetic substances, which, in concentrations appropriate vehicles, of which the final purpose is to afford fragrance to persons, objects or environment, including the extracts, toilet water, the creamy perfumes, the bath preparations and environmental fragrance, manufactured in liquid and gelled, viscous or solid form.

These kinds of compositions usually uses the ethylic alcohol in view of its refreshing benefits drying time, volatilization, preservation, solubility, inter alias.

However, the commonly used ethylic alcohol, in spite of its beneficial features for this sort of formulation, shows a characteristic and pronounced odor, and may affect the olfactory perception of the products, and, for several times, releasing an odor characterized as piquant or pungent. As far as the perfumes, deodorants, after-shaving lotions and other sorts of formulation are concerned, where the concentration of ethanol is considerable, possibly reaching up to 90%, the ethanol conventionally used may interfere with the performance of the fragrances. This may imply an olfactory perception less pleasant than the odor shown by the fragrance in its pure state, owing to the perception together with the piquant or pungent odor of the ethanol.

As the ethanol is an ingredient not easily replaceable with other solvents in its applications, it is highly used, in spite of its olfactory inconveniences.

In this area, there is the particular interest in using a type of alternative ethanol, enabling the development of formulations presenting the drying benefit of the volatility and suitable refreshment, without showing the inconvenient pungent odor, which is a feature of the conventional ethanol and, further, where the ethanol may contribute to making the olfactory perception more pleasant, through perfectly combining the fragrance and other base components, composing a different product. Additionally, this alternative ethanol, when used in personal care products, cosmetics or perfumes, should not cause allergic or irritations on the skin and mucous membranes, which would make unfeasible its use. The combination of these features represents the built-in art in the development of this sort of application.

Document EP0196340B1 protects a cosmetic composition containing conventional ethanol through the contact with a solid residue of alcoholic fermentation of grapes, obtained from the producing process of wine. It discloses, as a major object, the removal of the pungent odor present in ethanol, and may, for this purpose, use the ethylic alcohol derived from the chemical synthesis or fermentation of several substrates. Differently from this invention, it does not make use of the vinic alcohol obtained from the fermentation of grapes which contributes with a specific odor for the olfactory perception of the compositions.

Patent JP61204116A2 protects the application of wines treated with calcium salts in deo-colognes and perfumes, with a view to masking the irritating odor of ethanol and, further, contributing to the moistening of the skin, owing to the presence of saccharides, amino acids and peptides. Differently from this invention, it does not use the vinic alcohol obtained from the grape fermentation, rather the wine in its full state, bringing along other molecules, which might injure the coloring and cleanliness of the final composition.

This invention discloses the application of a specific variation of ethanol (ethylic alcohol) in personal care products, perfumes and cosmetics. The compositions derived from this invention contain, combined with this specific ethanol, conventionally used ingredients for the formulation of products having distinct purposes. The ethanol may be used for the purpose of promoting the volatility of the fragrance for making feasible the olfactory perception, or, then, performing the functions of promoting the sensation of refreshment, astringency, cleanliness or acting on the preservation of the formulation, or as a solubilizer.

The ethanol conventionally used in the cosmetic industry for personal care products, derives mainly from the fermentation of vegetables rich in sugars and amides, mainly sugar cane, beet, corn, rice, among other cereals. Over the time of fermentation, there may occur the formation of by-products, such as glycerin and the organic acids. In the personal care product industry, cosmetics and perfumes, the alcohol is normally purified in order to prevent influence from these by-products in the final product.

Another way of the conventional production of ethanol is the synthetic one, from acetylene or ethylene derived from petroleum by a gaseous synthesis (CO = O),among other mechanisms of synthesis.

This invention mainly features the innovating application of the vinic alcohol, partially or totally replacing the conventional ethylic alcohol in personal care products, cosmetics or perfumes. The application of the vinic alcohol is known and commonly used in the alcoholic beverage industry; one of the main applications is that of adjusting the alcoholic degree of the beverages. The vinic alcohol is the ethylic alcohol resulting from the fermentation of only grapes (Vitis vinifera sp) wherein there occurs the transformation of the sugars by microorganisms, resulting in a must suffering a later distillation. At this stage, in view of the fact of the must components presenting different boiling points, the first steams are always produced by more volatile elements, releasing from the original liquid mass. This is the case of the ethylic alcohol, carrying along molecules characterizing the material used as a fermentation source or by-products. This feature makes the vinic alcohol an interesting product from the olfactory point of view, making it different from the alcohol derived from other biological or synthetic sources, resulting in a pleasant olfactory combination, when harmonizing with the fragrances and other raw materials of the product base.

The vinic alcohol, by way of example, was applied to cologne deodorant in comparison with the conventional ethylic alcohol, within the following base formulation:

| Ingredients | Conventional Formulation A (%m/m) | Formulation B Object of this Invention (%m/m) |
|---|---|---|
| Fragrance | 10,00 | 10,00 |
| Water | 10,00 | 10,00 |
| Triclosane | 0,05 | 0,05 |
| HT | 0,05 | 0,05 |
| Benzophenone-2 | 0,10 | 0,10 |
| Sugar cane ethylic alcohol | Qsp 100 | - |
| Vinic alcohol | - | Qsp 100 |

These prototypes have been submitted to a triangular evaluation, performed by a panel of specialized technicians in olfactory evaluations. The statistic analysis of the results was carried out on the basis of the binomial distribution and the results are depicted in the table below:

| Perception of the difference by the technicians | Frequency (total of 23 technicians) | % |
|---|---|---|
| Yes | 21 | 91 |
| No | 2 | 9 |

After evaluating the results of this panel, it was concluded that the combination of vinic alcohol with the fragrance showed an easy perceptible difference in relation to the use of the sugar cane ethylic alcohol. Further, the pungent odor of the ethanol was less noticed in the vinic alcohol-containing formulation.

lpso facto, the present invention does not offer only an application of ethylic alcohol with a view to improving volatilization of the fragrance, affording refreshment, preservation, solubilization of ingredients, cleanliness, astringency, inter alias. The invention also discloses the innovating application of a specific ethylic alcohol, the vinic alcohol, having differentiated olfactory features, in combination with the olfactory features in the fragrance and other base raw materials, in the formulation of personal care products, perfumes and cosmetics. It further shows a conceptual important contribution for the development and disclosure of products, as it acts as a link between the actual technical benefits and the pleasant aspect of the art of production of wines, exploited and directed to the consumers of personal care products, cosmetics and perfumes.

The compositions disclosed in this invention are obtained from the incorporation of vinic alcohol in concentrations from 0,1% to 99% in bases of personal care products, cosmetics or perfumes. Its use may be further combined with the ethylic alcohol resulting from other sources, such as fermentation of other substrates or chemical synthesis.

A fragrance, aromatic composition of natural and/or synthetic ingredients or essential oils from plants, may be incorporated in concentrations from 0,01% to 30% by weight relating to the total weight of the composition.

Preserving substances may be included, according to this invention.

Vitamins, such as tocopherol or tocopherol acetate, retinol palmitate or other active ingredients, such as antioxidant agents, anti-inflammatory and vegetable extracts may further be included with a view to promoting the skin treatment.

For protecting the compositions from oxidation, antioxidant agents may be added. As examples, we may cite hydroxytoluene, hydroxyanisol butyl (BHT), vegetable antioxidants or others, or the blending of the same.

The obtainment of the additional property of solar protection of the formulation of the invention is managed through the presence of inorganic pigments or the inclusion of a set of photoprotector organic components, such as p-methoxycyanamate of 2-ethylexyl, butylmetoxydibenzoilmethane, or others and blends of the same, properly solubilized in their suitable solvents.

The use of conventional ethanol may be performed, provided that partially combined with the vinic alcohol.

Components of an oily nature, such as mineral oils, their fractions and by-products, hydrocarbons glycerides, esters and cosmetic ethers, silicones, elastomers, Guebert alcohols, vegetable oils, acids and fatty alcohols, among others and mixtures of the same, may also be used, as well as surfactants, such as emulsifiers or solubilizers.

Thickening agents, such as gums from vegetable biotechnological origins, acrylates, styrene polymers, celluloses and mixtures of the same, among others, may be used.

Moistening ingredients and humidifiers, such as glycols, polysaccharides, proteins or fractions of the same, among others, may be used.

Coloring substances may be added for modifying the aspect of the product.

This invention may further contain suspended particles, such as iron oxide, mica, polyethylene terephthalate (glitters), silica derivatives, titanium bioxide, zinc oxide, among others, as well as the respective mixtures, with a view to protecting the skin or mucous membranes and further as for ornaments.

The following instances illustrate the invention. The materials are combined and the amounts are represented in percentage by weight, on the basis of the total weight of the composition.

### Example I:

| Cologne Deodorant | Concentration |
|---|---|
| Fragrance or essential oils | 2.0%-18.0% |
| Solar Filter | 0.01% - 1.0% |
| Antioxidant | 0.01% - 0.5% |
| Preservative (triclosane or others) | 0.05% - 0.3% |
| Water | 8.0% - 20% |
| Vinic alcohol | 40% - 90% |

### Example II

| Gelled Solar Protector | Concentration |
|---|---|
| Fragrance or essential oils | 0.01%-30% |
| Antioxidant | 0.01% - 1% |
| Solar filter | 0.01%-25% |
| Vitamins, vegetable extracts or other active ingredients | 0.01%-3% |
| Humidifiers | 0.01%-10% |
| Water | 0.1%-20% |
| Vinic Alcool | 1%-90% |
| Ethylic alcohol from sugar cane | 1%-90% |

This invention is not limited to the representation herein commented on or illustrated, and must be understood in its broad scope. Many modifications and other embodiments of the invention will come to the mind of those skilled in the art, to which this invention belongs, having the benefit of the teaching submitted in the previous descriptions. Moreover, it is to be understood that the invention is not restricted to the specific form exhibited and that modifications and other forms are understood as incorporated into the scope of the claims attached. Even though specific terms are used herein, we have done so in a generic and descriptive manner and not for a purpose of restriction.

## Claims

1. "USE OF VINIC ALCOHOL IN PERSONAL CARE PRODUCTS, COSMETICS AND PERFUMES", composition for personal care products, cosmetics and perfumes showing specific olfactory features, **characterized by** presenting a basic formulation, with contents by weight of product from 0,1% to 90% of vinic alcohol.

2. "USE OF VINIC ALCOHOL IN PERSONAL CARE PRODUCTS, COSMETICS AND PERFUMES", composition for personal care products, cosmetics and perfumes of claim 1, **characterized by** the presence of conventional ethylic alcohol, combined with vinic alcohol.

3. "USE OF VINIC ALCOHOL IN PERSONAL CARE PRODUCTS, COSMETICS AND PERFUMES", composition for personal care products of claims 1 and 2, **characterized by** presenting a basic formulation containing vinic alcohol, in contents preferably between 1% and 60% by weight of the product.

4. "USE OF VINIC ALCOHOL IN PERSONAL CARE PRODUCTS, COSMETICS AND PERFUMES", composition of cosmetic products of claims 1 and 2, **characterized by** presenting a basic formulation containing vinic alcohol in contents preferably between 1% and 90% by weight of product.

5. "USE OF VINIC ALCOHOL IN PERSONAL CARE PRODUCTS, COSMETICS AND PERFUMES", composition of perfumes of claims 1 and 2, **characterized by** presenting a basic formulation containing vinic alcohol in contents preferably between 10% and 90% by weight of product.
